# EUROPEAN PATENT APPLICATION

(11) **EP 2 322 100 A1**
(43) Date of publication of application: **18.05.2011**
(21) Application number: 09813034.7
(22) Date of filing: 03.09.2009
(51) Int. Cl.: A61B 8/06

(54) **ULTRASOUND IMAGE DISPLAY APPARATUS AND ULTRASOUND IMAGE DISPLAY METHOD**

(30) Priority: 09.09.2008 JP 2008231312
(71) Applicant: Olympus Medical Systems Corp., Tokyo 151-0072 (JP)
(72) Inventor: ICHIKAWA Junichi, Tokyo 151-0072 (JP)
(74) Representative: Gunzelmann, Rainer
(86) International application number: PCT/JP2009/065435
(87) International publication number: WO 2010/029888

(57) **Abstract**

An ultrasound diagnostic apparatus 1 includes a control section 13 that performs control such that an ROI is displayed on a screen 21 upon input of a predetermined command. The ROI is for displaying a flow mode image of a region including a start point and an end point of a puncture path of a puncture needle 2c that appears on a screen of an ultrasound image when the puncture needle 2c is protruded from an ultrasound probe 2a.

## Description

### Technical Field

The present invention relates to an ultrasound image display apparatus and a method for displaying an ultrasound image, and particularly to an ultrasound image display apparatus and a method for displaying an ultrasound image which can display a flow mode image

### Background Art

Conventionally, ultrasound diagnostic apparatuses are widely used in the medical field and so on. An ultrasound diagnostic apparatus obtains ultrasound data by transmitting ultrasound from a distal end portion of an ultrasound probe to an object to be examined and receiving the ultrasound therefrom to generate an ultrasound image. By observing such an ultrasound image, physicians and others can noninvasively diagnose the inside of the object to be examined.

Among the ultrasound probes to be connected to an ultrasound diagnostic apparatus is an ultrasound probe that can protrude a puncture needle or the like for biopsy from the distal end portion thereof. Moreover, some ultrasound diagnostic apparatuses have a so-called flow mode and can display blood flow when used for the diagnosis of the inside of a body cavity. In a flow mode of a color flow or a power flow, it is possible to detect blood flow by utilizing Doppler effect and display a blood vessel in an ROI (Region Of Interest). Therefore, a physician can make the puncture needle protrude from the distal end portion of a probe to perform biopsy while observing an ultrasound image.

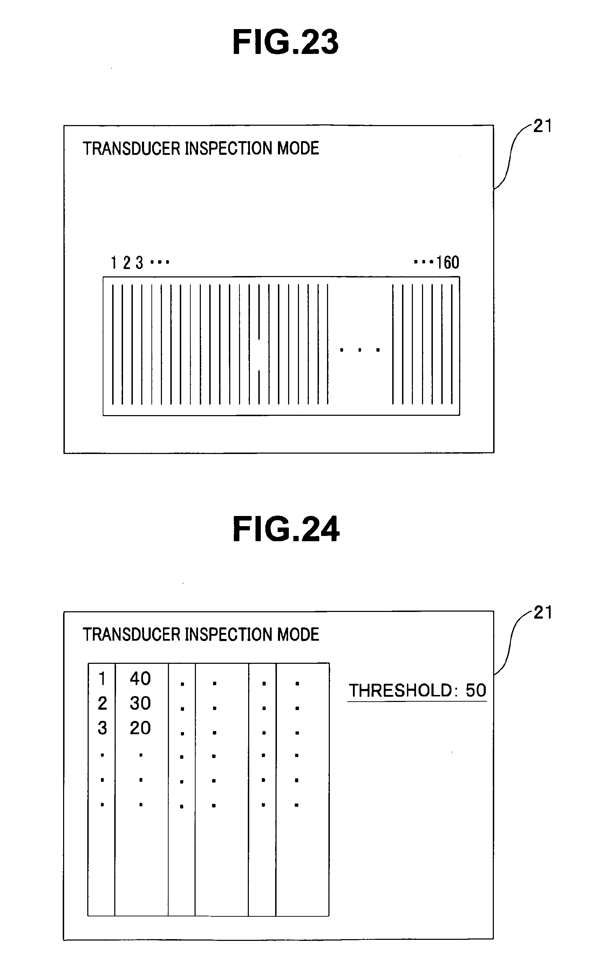

Usually, such an ROI is preset at a position convenient for observing a lesioned part, for example, a position of 6 o'clock direction with respect to the probe, and Japanese Patent Application Laid-Open Publication No. 2007-268148 proposes an ultrasound diagnostic apparatus that can change the position of the ROI on the screen. Fig. 24 of the publication discloses an ultrasound diagnostic apparatus in which the position of an ROI is designated to a position of a puncture needle guide image.

However, although the proposed apparatus can designate the position of an ROI to the position of a puncture needle guide image, the ROI is not set so as to include all the paths, that is, routes through which the puncture needle passes. Therefore, it is impossible to confirm the presence of a blood vessel in a region which is outside the ROI and through which the puncture needle passes.

Therefore, the present invention has been made in view of the above described points, and has its object to provide an ultrasound image display apparatus that displays the position of an ROI so as to cover all the paths through which the puncture needle passes.

### Disclosure of Invention

### Means for Solving the Problem

According to an aspect of the present invention, there is provided an ultrasound image display apparatus, comprising a display control section that performs control such that a flow mode image display region is displayed on a screen of an ultrasound image, wherein the flow mode image display region is for displaying a flow mode image of a region including a start point and an end point of a puncture path of a puncture needle that appears on the screen when the puncture needle is protruded from an ultrasound probe.

According to an aspect of the present invention, there is provided a method for displaying an ultrasound image, comprising performing control such that a flow mode image display region is displayed on a screen of an ultrasound image, wherein the flow mode image display region is for displaying a flow mode image of a region including a start point and an end point of a puncture path of a puncture needle that appears on the screen when the puncture needle is protruded from an ultrasound probe.

### Brief Description of the Drawings

Fig. 1 is a configuration diagram showing a configuration of an ultrasound diagnostic apparatus including an ultrasound endoscope relating to a first embodiment of the present invention;
Fig. 2 is a diagram for illustrating an example of an ultrasound probe including a convex scanning type ultrasound transducer, relating to the first embodiment of the present invention;
Fig. 3 is a diagram showing an example of a GUI relating to the first embodiment of the present invention;
Fig. 4 is a diagram showing an example of an image designation screen relating to the first embodiment of the present invention;
Fig. 5 is a diagram to illustrate the correspondence between the operation and the display of the switching button, relating to the first embodiment of the present invention;
Fig. 6 is a diagram to illustrate the display state of an ultrasound image on a screen corresponding to the operation of the switching button, relating to the first embodiment of the present invention;
Fig. 7 is a diagram to illustrate a linear MPR display, relating to the first embodiment of the present invention;
Fig. 8 is a diagram showing an example of a cross-sectional display screen that displays each cross-section when performing linear MPR display, relating to the first embodiment of the present invention;
Fig. 9 is a diagram showing another example of a cross-sectional display screen that displays each cross-section when performing linear MPR display, relating to the first embodiment of the first invention;
Fig. 10 is an example of screen display during a normal display, relating to the first embodiment of the present invention;
Fig. 11 is an example of screen display during an enlarged display, relating to the first embodiment of the present invention;
Fig. 12 is a diagram showing an example of a screen in which a gray scale bar and a color scale bar are displayed along with an image, relating to the first embodiment of the present invention;
Fig. 13 is a diagram showing another example of a screen in which a gray scale bar and a color scale bar are displayed along with an image, relating to the first embodiment of the present invention;
Fig. 14 is a diagram to illustrate a display range designation screen during an enlarged display of ultrasound image, relating to the first embodiment of the present invention;
Fig. 15 is a diagram showing the display position of an ROI during the observation of a digestive tract, relating to the first embodiment of the present invention;
Fig. 16 is a diagram showing the display position of an ROI during the observation of a respiratory organ, relating to the first embodiment of the present invention;
Fig. 17 is a diagram to illustrate the movement of an ROI, relating to the first embodiment of the present invention;
Fig. 18 is a diagram to illustrate the movement of an ROI, relating to the first embodiment of the present invention;
Fig. 19 is a diagram to illustrate an example of automatic adjustment of the position and size of ROI, relating to the first embodiment of the present invention;
Fig. 20 is a diagram to illustrate the case when a comment is inputted on the screen, relating to the first embodiment of the present invention;
Fig. 21 is a diagram showing another example of the display position of an annotation list display section, relating to the first embodiment of the present invention;
Fig. 22 is a diagram to illustrate the setup of a comment input position with a cursor, relating to the first embodiment of the present invention;
Fig. 23 is a diagram showing an example of the screen to be displayed on a monitor when the ultrasound diagnostic apparatus 1 is turned into a transducer inspection mode, relating to the first embodiment of the present invention;
Fig. 24 is a diagram showing another example of the screen to be displayed on a monitor when the ultrasound diagnostic apparatus 1 is turned into a transducer inspection mode, relating to the first embodiment of the present invention;
Fig. 25 is a diagram showing an example of the screen that displays a detected voltage of power supply, relating to the first embodiment of the present invention;
Fig. 26 is a diagram showing an example of message display, relating to the first embodiment of the present invention;
Fig. 27 is a diagram showing an example of a setup screen of probe frequency, relating to the first embodiment of the present invention;
Fig. 28 is a diagram showing an another example of the setup screen of probe frequency, relating to the first embodiment of the present invention;
Fig. 29 is a diagram showing an example of a gain setup table of B-mode relating to the first embodiment of the present invention;
Fig. 30 is a flowchart showing an example of the flow of processing including the decision processing of the size and position of ROI, relating to the first embodiment of the present invention;
Fig. 31 is a flowchart showing an example of the flow of the extraction processing of the puncture needle in Fig. 30;
Fig. 32 is a diagram showing an example of acquired image, relating to the first embodiment of the present invention;
Fig. 33 is a diagram showing an example of a generated image of a puncture needle, relating to the first embodiment of the present invention;
Fig. 34 is a flowchart showing an example of the flow of the decision processing of ROI in Fig. 30;
Fig. 35 is a diagram to illustrate the centerline of a puncture needle, and the width and length of an ROI, relating to the first embodiment of the present invention;
Fig. 36 is a diagram showing a determined ROI relating to the first embodiment of the present invention;
Fig. 37 is a diagram showing an example of a sector-shaped ROI, relating to a second embodiment of the present invention; and
Fig. 38 is a flowchart showing an example of the flow of processing including the decision processing of ROI of an ultrasound diagnostic apparatus relating to the second embodiment of the present invention.

### Best Mode for Carrying Out the Invention

Hereafter, embodiments of the present invention will be described by using drawings.

### (First Embodiment)

### 1. System configuration

Hereafter, a configuration of an ultrasound diagnostic apparatus relating to a first embodiment of the present invention will be described.

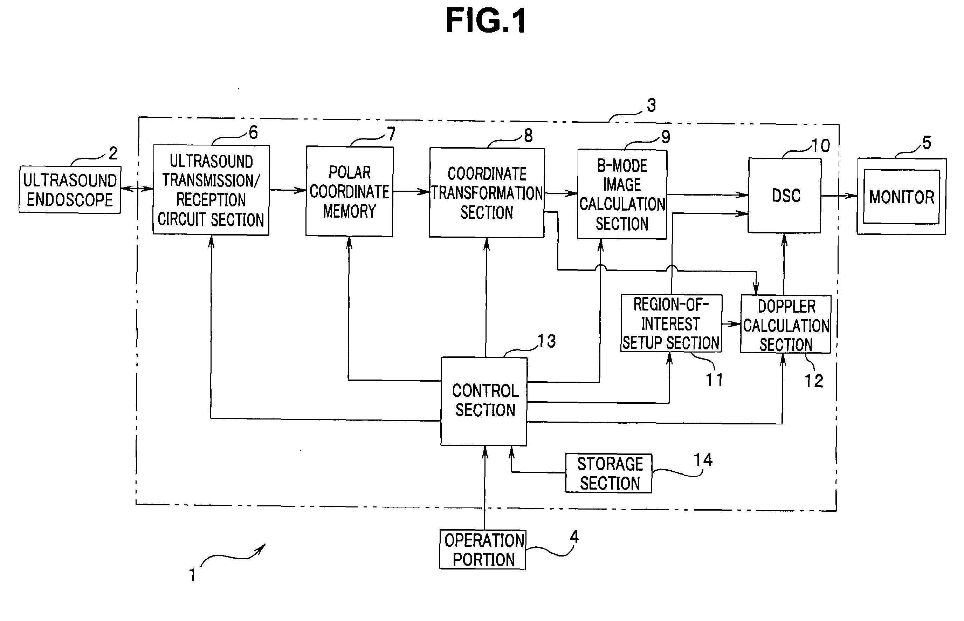

Fig. 1 is a configuration diagram showing a configuration of an ultrasound diagnostic apparatus including an ultrasound endoscope relating to the first embodiment of the present invention.

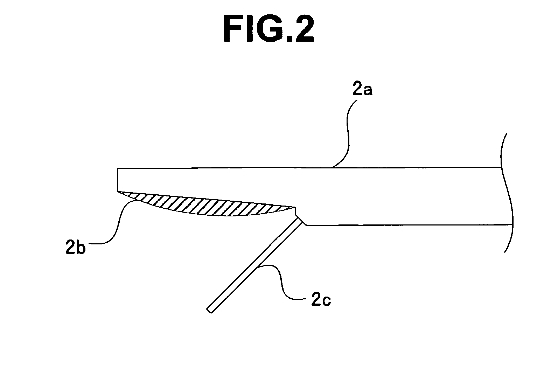

As shown in Fig. 1, an ultrasound endoscope apparatus 1 that makes up an ultrasound image processing apparatus of the first embodiment of the present invention is configured to include: an ultrasound endoscope 2 as ultrasound detection means that includes an ultrasound transducer (see Fig. 2) for transmitting/receiving ultrasound to and from a subject in an ultrasound probe located at the distal end of an insertion portion of the endoscope; an ultrasound observation apparatus 3 as an ultrasound image display apparatus that drives an ultrasound transducer of the ultrasound endoscope 2 and generates an ultrasound image from an echo signal; and an operation portion 4 as Region-Of-Interest designating means that instructs various operations to the ultrasound observation apparatus 3. An observation monitor 5 for displaying an ultrasound image or the like is connected to the ultrasound observation apparatus 3. It is noted that the monitor 5 is of a touch panel type. Of course, the monitor 5 may be a CRT, a liquid crystal monitor, and the like.

The ultrasound observation apparatus 3 is configured to include: an ultrasound transmission/reception circuit section 6 as ultrasound transmission/reception means that outputs a driving signal to drive an ultrasound transducer and transforms an echo signal from the ultrasound transducer into polar coordinate data; a polar coordinate memory 7 that stores the polar coordinate data from the ultrasound transmission/reception circuit section 6; a coordinate transformation section 8 that transforms the polar coordinate data of the polar coordinate memory 7 into Cartesian coordinate data; a B-mode image calculation section 9 as first mode image calculating means that generates B-mode image data based on the output data of the coordinate transformation section 8; a DSC (Digital Scan Converter) 10 that generates ultrasound image data in which the blood flow information data and the B-mode image data are combined by performing coordinate transformation and interpolation processing based on blood flow information data calculated at a below-described Doppler calculation section 12 and B-mode image data calculated at the B-mode image calculation section 9 so as to conform to a scan shape of the ultrasound transducer, and outputs the generated ultrasound image data to the monitor 5; a Region-Of-Interest setup section 11 that sets a region of interest (hereafter abbreviated as ROI) on the B-mode image data and outputs the data of the ROI to the DSC 10 and a Doppler calculation section 12; the Doppler calculation section 12 as second mode image calculating means that converts blood flow information in the ROI into color data and outputs the color data to the DSC 10; a control section 13 that includes a CPU and controls the above-described each section; a storage section 14 that stores below-described various setup data and processing programs in addition to image data.

The ultrasound diagnostic apparatus 1 is configured to include a computer apparatus including a CPU. The computer apparatus executes an application program (hereafter, simply referred to as application) for implementing the function of ultrasound diagnostic apparatus or ultrasound image display apparatus on an operating system (hereafter referred to as OS) of, for example, Windows (registered trademark).

It is noted that while the application of the ultrasound diagnostic apparatus 1 is executed on such an OS, the program of a file management system on the OS, for example, "Exploler" is disabled for execution in order for preventing erroneous setup of various setup data and the like. To be specific, when log-in is performed by a user, the application of the ultrasound diagnostic apparatus is configured to start up immediately in order for disabling such file management system for execution.

Further, the application of the ultrasound diagnostic apparatus 1 is operated under a dedicated OS mode, and not only the execution of Exploler is disabled as described above, but also the function of a tool that is capable of file operation, such as a start menu display, is disabled. Similarly, various types of shortcut keys on the OS are also disabled. Further, the auto-run functions of a MOD, a USB memory, an HDD, a flash memory reader, and a CD are disabled as well. Thus, configuration is such that a USB keyboard and a USB mouse are not recognized while the application of the ultrasound diagnostic apparatus 1 is activated.

Moreover, it is configured such that not only an electronic scanning type ultrasound endoscope, but also a mechanical scanning type ultrasound endoscope is connectable to the ultrasound diagnostic apparatus 1, and sensitivity quantification software and noise quantification software, which are diagnostic software, are made compatible with both the data of mechanical scanning type ultrasound endoscope and the data of electronic scanning type ultrasound endoscope.

### 2. Ultrasound probe

Fig. 2 is a diagram for illustrating an example of an ultrasound probe that can protrude a puncture needle from a distal end portion of the ultrasound endoscope 2 and includes a convex scanning type ultrasound transducer. It is noted that although not shown in Fig. 2, the ultrasound probe is also provided with a transducer for radial scanning.

As shown in Fig. 2, a convex scanning type ultrasound transducer 2b is provided at the distal end portion of the ultrasound probe (hereafter, simply referred to as the probe) 2a. Then, the distal end portion is configured such that a puncture needle 2c for biopsy can be protruded from a predetermined position, and the puncture needle 2c can be used to take a biological tissue sample.

### 3. Image display

### 3.1 Display mode

The ultrasound diagnostic apparatus 1 as an ultrasound image display apparatus has two display modes of a B-mode and a flow mode, and the flow mode includes two modes of a color flow mode and a power flow mode. Ultrasound images according to such modes are displayed on the screen of the monitor 5.

Switching of the display mode by a user is performed by operating a B-mode button, a color flow mode button (hereafter, referred to as a CFM button), and a power flow mode button (hereafter, referred to as a PFM button) on the operation portion 4.

It is noted that in the present embodiment, the number of levels of flow gain is, for example, not less than 30.

Upon the B-mode button being depressed, the display mode turns into a B-mode and a B-mode image is displayed. Upon the CFM button being depressed in a B-mode state, the display mode is changed to a color flow mode, and a color flow mode image is displayed. Similarly, upon the PFM button being depressed in a B-mode state, the display mode is changed to a power flow mode and a power flow mode image is displayed.

Moreover, upon the B-mode button being depressed in a color flow mode state, the display mode is changed to the B-mode, and upon the PFM button being depressed, the display mode is changed to the power flow mode.

Similarly, in a power flow mode state, upon depression of the B-mode button, the display mode is changed to the B-mode, and upon depression of the CFM button, the display mode is changed to the color flow mode.

It is noted that upon the CFM button being depressed in a color flow mode state, the display mode is changed to the B-mode, and in a power flow mode state as well, upon the PFM button being depressed, the display mode is changed to the B-mode. Even if the B-mode button is depressed in a B-mode state, the display mode remains in the B-mode.

### 3.2 Record and replay of image

Moreover, the ultrasound diagnostic apparatus 1 can execute the function of recording and releasing images while generating an ultrasound image. Recording means that data of ultrasound image is saved as digital data in the storage section 14, and releasing means that an ultrasound image is outputted to a printer not shown, or filed in a filing apparatus via a network. The data format of image data at the time of recording may be a bit map format, a JPEG format, or any dedicated format.

It is noted that while a two-dimensional image is being generated, even if recording operation for recording is performed, or releasing operation for printer output, etc. is performed, both operations are enabled, but while image data is acquired and a three-dimensional image data is being generated, recording operation is enabled, but releasing operation is not enabled.

Further, it is noted that a screen to be displayed on the monitor 5 during observation can be saved as data of a bitmap format (bmp format) or a JPEG format in the storage section 14 by a screen capture function.

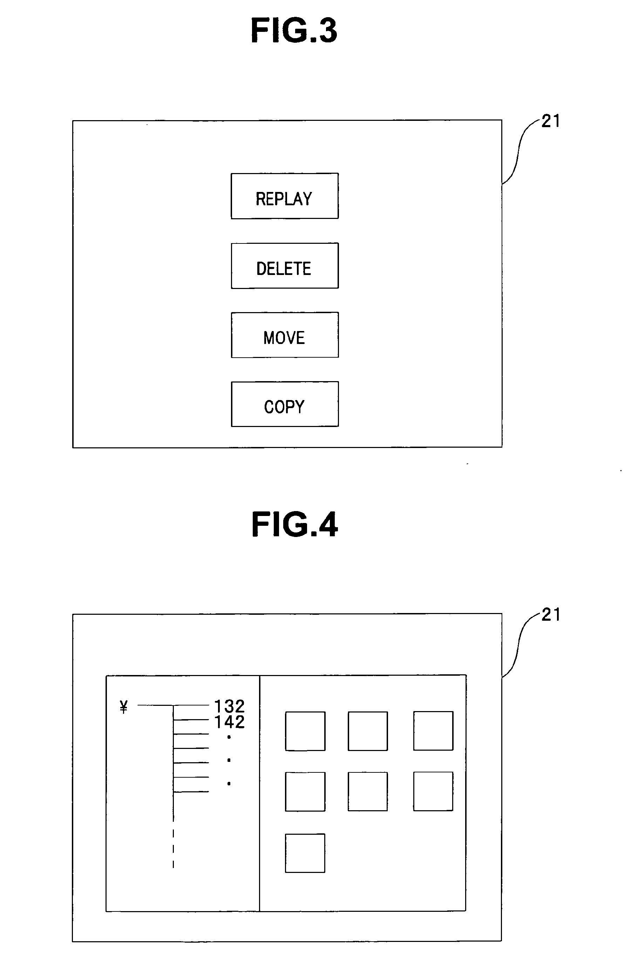

Replay of recorded image data, and the like are performed via a graphical user interface (hereafter, referred to as a GUI). Fig. 3 shows an example of the GUI. When performing the replaying, deleting, moving or copying of acquired image data, a user can cause the screen of Fig. 3 to be displayed on the screen 21 of the monitor 5 by such as depressing a data base key on the operation portion 4. For example, when a user wants to replay a certain image, the user touches and selects a replay button on the screen 21. Therefore, Fig. 3 is a dialog screen for selecting functions such as replaying and the like.

For example, upon the replay button being selected, the screen is changed to the screen of Fig. 4. Fig. 4 is a diagram showing an example of an image designation screen. In Fig. 4, on the left side is a tree display section that displays the configuration of a folder in a tree format, and on the right side is a thumbnail display section that displays thumbnail images of the image data included in the folder.

The user selects a folder that includes the screen that he/she wants to replay, in the tree display section on the screen 21 of Fig. 4, and selects a thumbnail image corresponding to the image that he/she wants to replay, in the thumbnail display section. Then, the image corresponding to the selected thumbnail image is replayed. Deletion, moving, and copying of images are performed in a similar manner. Therefore, Fig. 4 can be said to be a dialog screen for selecting a file. Thus, using the two screens of Figs. 3 and 4 as a GUI makes it possible to allow intuitive grasping of operations and to improve the operability of user.

It is noted that in Fig. 3, "MOVE" is a button for executing the processing to move file data to another folder, etc. Upon the "MOVE" button being selected, a CPU of the control section 13 confirms available capacity of the destination folder, that is, the shipping destination folder, and executes "MOVE" processing when the available capacity is not less than a capacity (CC + CD) that is the sum of the capacity (CC) of the file to be moved and a certain amount of margin (CD).

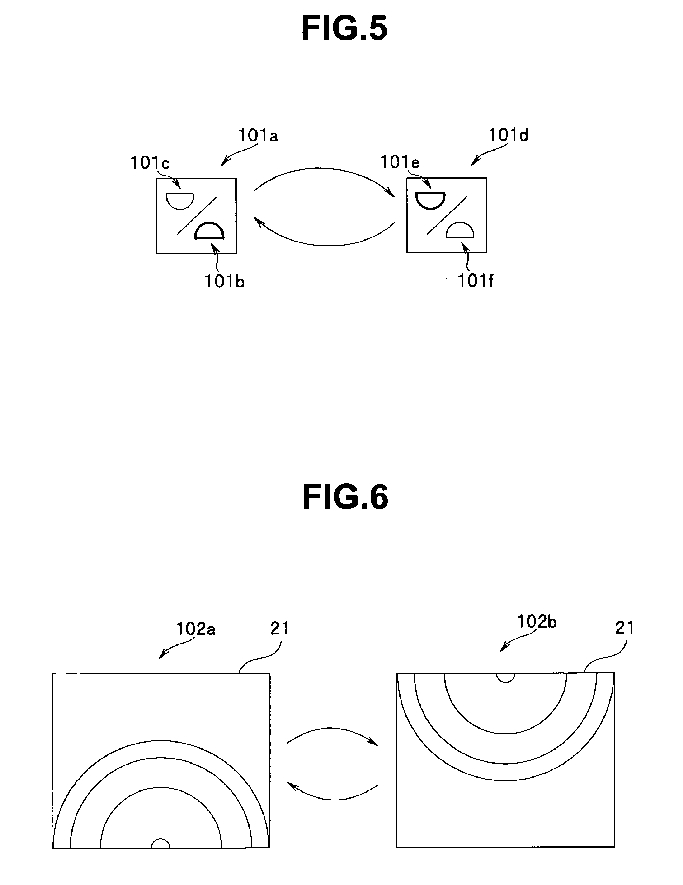

A touch panel is incorporated into the operation portion 4, and various commands can be displayed on the screen of the touch panel. For example, an image of switching buttons 101 of upper and lower half displays for performing the switching between an upper half display and a lower half display of an ultrasound image of radial scanning is displayed on the screen of the touch panel. As a user touches once a switching button 101 a as shown in Fig. 5, the displays of upper half and lower half displayed on the screen 21 is switched.

Fig. 5 is a diagram to illustrate the correspondence between the operation and the display of the switching button. Fig. 6 is a diagram to illustrate the display state of an ultrasound image on the screen 21 corresponding to the operation of the switching button. In a display state 101a of Fig. 5, a picture that indicates an upper half display of a full circle ultrasound image, that is, an icon 101b is displayed in a predetermined color, for example, green and a picture 101 c that indicates a lower half display is displayed in a predetermined color, for example, white. In a display state 101d, a picture 101e that indicates a lower half display is displayed in the same predetermined color as that of the picture 101b, and a picture 101f that indicates an upper half display is displayed in white. It is noted that in Fig. 5, thick lines indicating the pictures 101b and 101e mean that the pictures are displayed in green.

When the display of the switching button of Fig. 5 is in the display state 101 a, an upper half ultrasound image display 102a is displayed on the screen of the monitor 5 as shown on the left side of Fig. 6, and when the display of the switching button is in the display state 101d, a lower half ultrasound image display 102b is displayed on the screen of the monitor 5 as shown on the right side of Fig. 6.

Thus, switching by the switching button on the screen 21 is a toggle switching operation, and an ultrasound image displayed on the screen 21 is alternately switched between an upper half and lower half image displays according to the switching of the switching button.

It is noted that although it is possible to separately set parameters such as focus position, scanning line density, etc. for the upper half and lower half ultrasound image displays 102a and 102b, the settings may be made in common and the same. Moreover, configuration may be such that the monitor 5 is made up of a touch panel, and a display corresponding to a touch panel of the operation portion 4 may be displayed on a part of the screen 21.

Moreover, the ultrasound diagnostic apparatus 1 also has a function of linear MPR display.

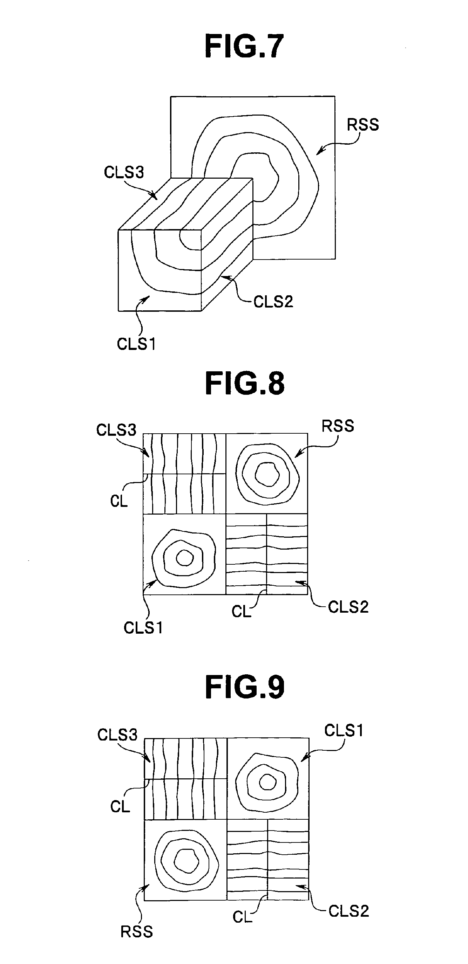

When the linear MPR display is performed, a reference position cross-section RSS and each cross-section along a cut line are displayed. Fig. 7 is a diagram to illustrate the linear MPR display. Figs. 8 and 9 are diagrams showing an example of a cross-sectional display screen that displays each cross-section when performing the linear MPR display.

Drawings of three cross-sections a CLS1, CLS2, and CLS3 at positions designated from the reference position cross-section RSS as shown in Fig. 7 are displayed on the screen as shown in Fig. 8.

In the cross-sectional display screen of Fig. 8, although usually a three-dimensional image is displayed in a frame in the upper right, the reference position cross-section RSS is in the upper right and the cross-section CLS1 parallel with the reference position cross-section RSS is displayed in the lower left, as shown in Fig. 8. The two cross-sections CLS2 and CLS3 are displayed in the lower right and the upper left, respectively. The two cross-sections CLS2 and CLS3 are displayed with a cut line superposed thereon, respectively.

Then, Fig. 9 is an example in which the cross-section CLS 1 is displayed in the upper right, and the reference position cross-section RSS is displayed in the lower left. It is possible to change over between the display forms of Fig. 8 and Fig. 9 by user setup.

It is noted that configuration may be such that nothing is displayed on the upper right during live, and the cross-section display screen of Fig. 9 is displayed immediately after freeze.

Moreover, the ultrasound diagnostic apparatus 1 also has a function of enlargement display.

If the display state is changed from a state of normal display to a state of enlarged display in a sate in which an ultrasound image is displayed, there may be a case in which the entire enlarged ultrasound image can not be displayed on the screen. That is, for example, the case in which even if the display range of an ultrasound image of normal display is 6 cm, the display range of enlarged display is not 6 cm. In order that a user may not misunderstand the display range in such a case, configuration is made such that the display range is displayed when enlarged display is performed.

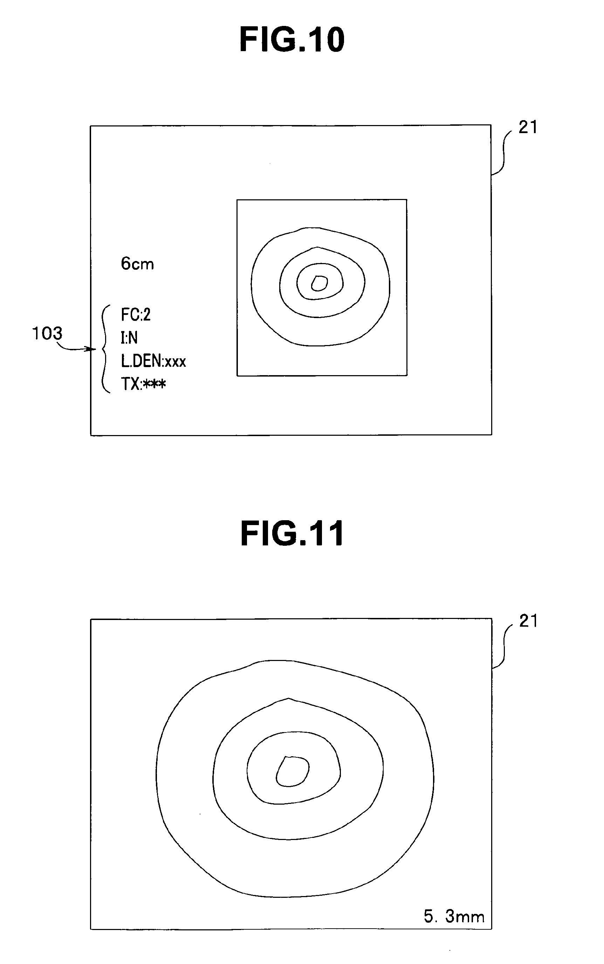

Fig. 10 is an example of screen display during a normal display. Fig. 11 is an example of screen display during an enlarged display. In the example of Fig. 10, although the display range is 6 cm during a normal display, the display range has become 5.3 cm as shown in Fig. 11 when the normal display is changed to an enlarged display, and therefore the display range thereof is displayed in the lower right of the screen. As a result, it is possible to prevent erroneous recognition of the display range by the user.

Moreover, during a normal display, setup data relating to an ultrasound image is displayed in the lower left on the screen 21. In Fig. 10, a number of frame correlation (FC), a gamma curve (I), a scanning line density (L. DEN), transmission power (TX) and the like are displayed as image quality data in an image quality display section 103.

It is noted that although such image quality display section 103 may be configured to be always displayed, the display of image quality display section 103 can be turned off by a user setup so as not to display.

Furthermore, during a flow mode, the gain of color flow is displayed by CFG, and the gain of power flow is displayed by PFG. For example, when the color flow gain is 10, the display will be "CFG:10" and when the power flow gain is 9, the display will be "PFG:9".

Moreover, the ultrasound diagnostic apparatus 1 has a display function of scale bar.

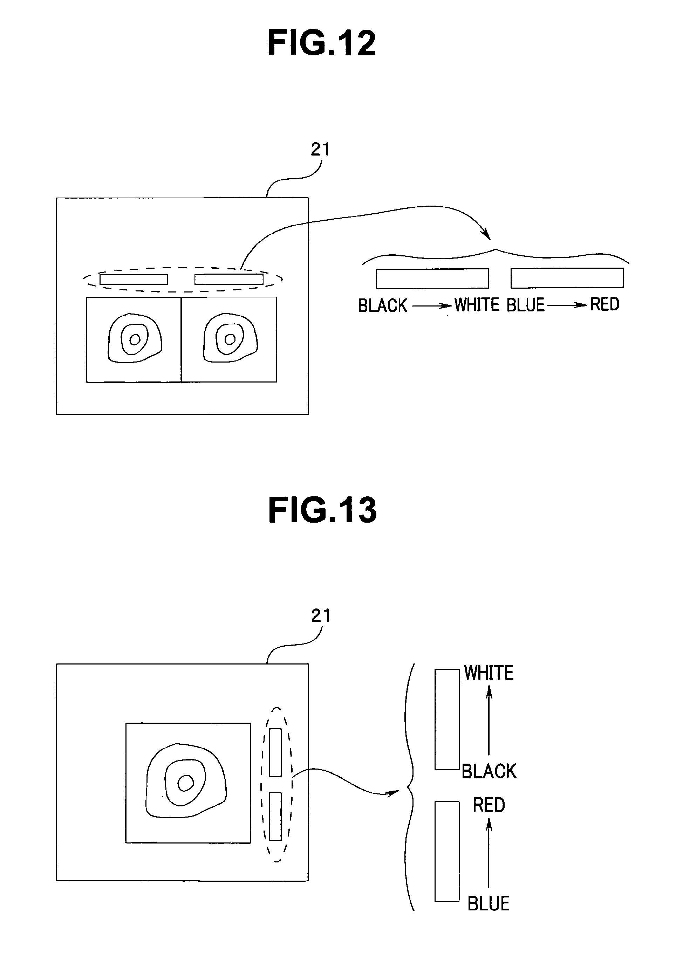

When an ultrasound image is displayed, a gray scale bar and a color scale bar are displayed respectively in conjunction with a number line. Figs. 12 and 13 are diagrams showing examples of screens in which a gray scale bar and a color scale bar are displayed respectively with an image.

In Fig. 12, each scale bar is laterally disposed, in which the gray scale bar indicates black to white from left toward right, and the color scale bar indicates blue to red from left toward right.

In Fig. 13, each scale bar is longitudinally disposed, in which the gray scale bar indicates black to white from bottom toward top, and the color scale bar indicates blue to red from bottom toward top.

Moreover, the ultrasound diagnostic apparatus 1 can change the display range of ultrasound image.

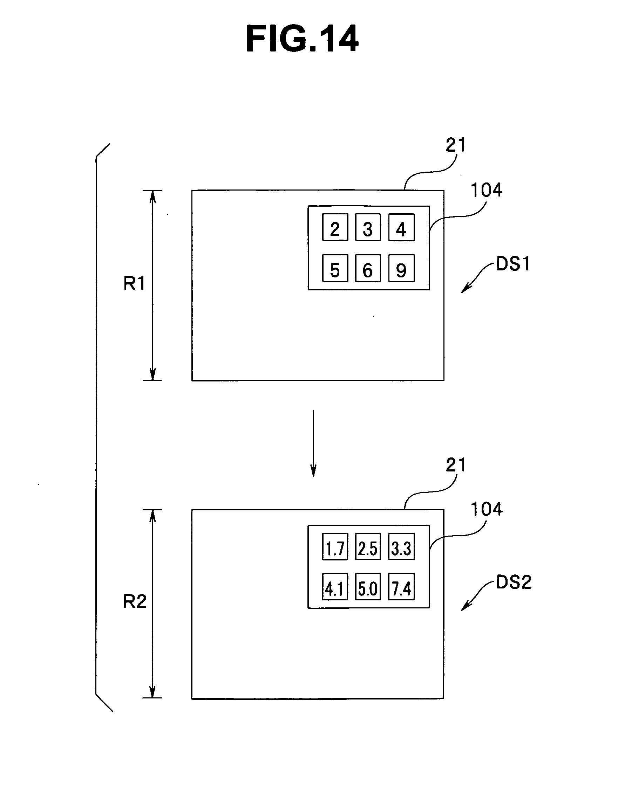

When an ultrasound image is enlarged, numerical values for designating the display range are changed as well according to the enlargement ratio. Fig. 14 is a diagram to illustrate a display range designation screen during an enlarged display of ultrasound image.

When the display range is changed from a state of R1 to an enlarged display mode, the numerical values of the display range in a display range designation display section 104 in an enlarged display state is changed as well according to the enlargement ratio as shown in Fig. 14. Thereby, the user can designate a display range of image according to the enlarged display state.

Moreover, the operation portion 4 is provided with a trackball. The function of the trackball is automatically changed according to operation states or display states.

That is, operating the trackball while an ultrasound image is displayed on the screen of the monitor 5 allows the user to perform image rotation, that is, a viewpoint change of displayed image.

Upon freeze of screen being instructed by an operation of a freeze button or the like when an image rotation function is assigned to the trackball, the image is freezed. Then, upon the trackball being operated in the freeze state, the function of the trackball is changed so as to be compatible with the function of interframe movement in a so-called cinememory, without being assigned to the function of image rotation. Upon the trackball being operated after the freeze, a frame image that is read out from the cinememory is displayed on the screen of the monitor 5.

It is noted that such change of the assignment of trackball function can be performed by a user setup. Therefore, changing the setup also makes it possible to configure such that even if freeze operation is performed when the function of image rotation is assigned to the trackball, the function of frame change of cinememory will not be assigned to the trackball, and the same assignment of the function of image rotation as before is maintained.

Further, it is also possible to configure such that the function of the trackball is compatible with the below described function of moving an ROI. The correspondence between the trackball and the movement of ROI will be described below.

### 4. ROI display

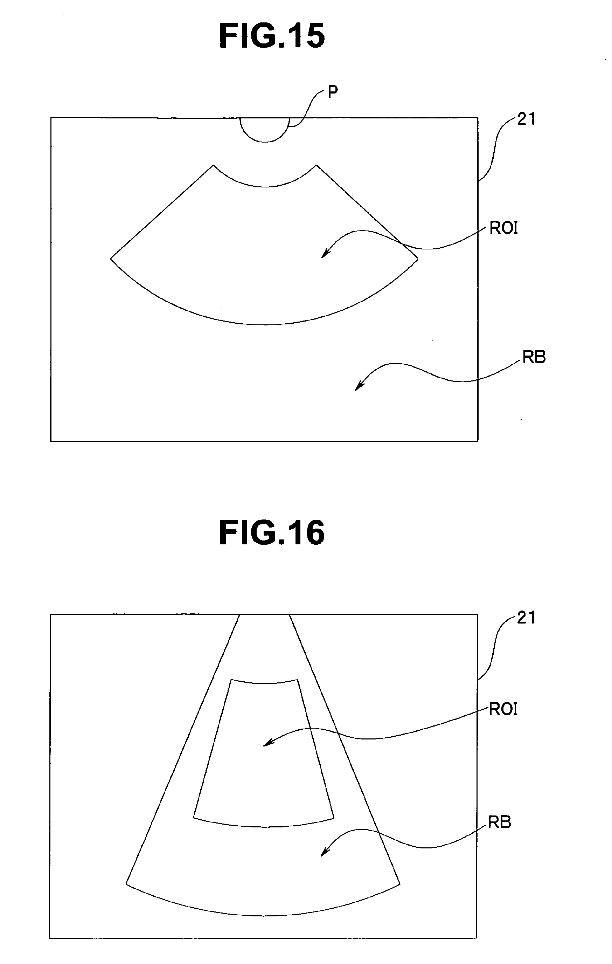

Usually, when ultrasound observation, etc. is performed by using a probe 2a, if an ROI is caused to be displayed on the monitor 5 prior to the observation, the ROI is displayed at a position set up by default as shown in Fig. 15 or 16. Fig. 15 is a diagram showing the display position of an ROI during the observation of a digestive tract. Fig. 16 is a diagram showing the display position of an ROI during the observation of a respiratory organ.

In the case of a digestive tract, the default position of ROI on the screen 21 of the monitor 5 is below a probe image P, that is, in the direction of 6 o'clock as shown in Fig. 15. The region outside the ROI is a region RB of B-mode display.

In the case of a respiratory organ, the default position of ROI on the screen 21 of the monitor 5 is in the B-mode display region RB, and in the so-called 6 o'clock direction as shown in Fig. 16. It is noted that the outside region around the B-mode display region RB is a region where no image is rendered.

Moreover, as described above, it is possible to make a change such that the function of the trackball is assigned to the function of moving an ROI, by the setup operation of a user. That is, it is made possible to move the position of an ROI on the screen by operating the trackball.

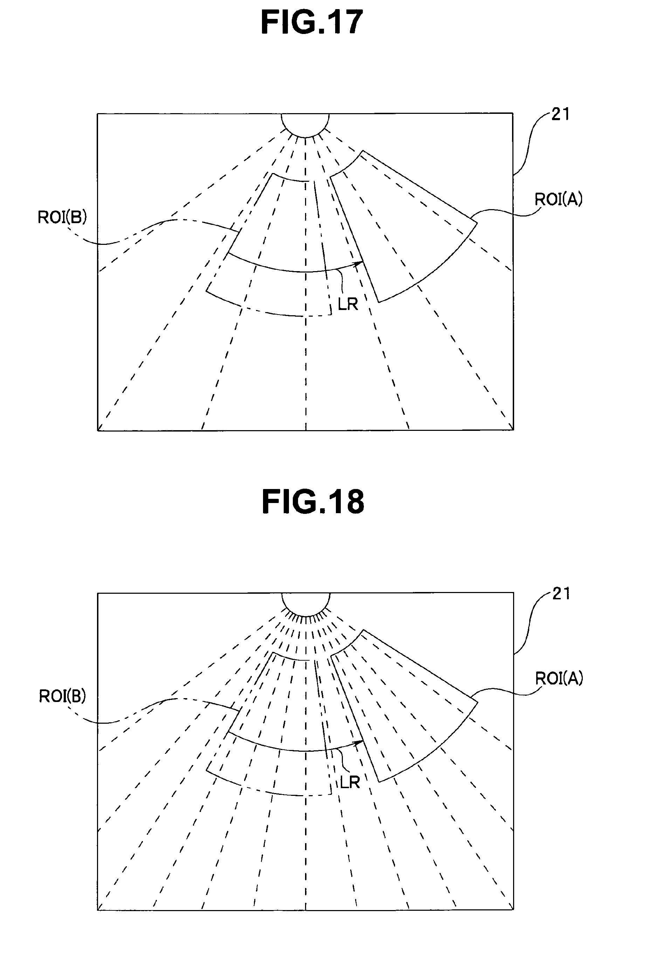

Figs. 17 and 18 are diagrams to illustrate the movement of an ROI, respectively. The sound ray density of the image of Fig. 17 is half of that of the image of Fig. 18. The ROI is moved on the screen by a user rotating the trackball, according to the amount of the rotation. In Figs. 17 and 18, an ROI(B) before movement and an ROI(A) after movement are displayed.

Here, the amount of movement of ROI is controlled such that the amount of movement of ROI on the screen according to the same amount of operation of the trackball becomes the same regardless of the sound ray density. In other words, although the sound ray density of the image of Fig. 17 is different from that of the image of Fig. 18, the ROI is moved on the screen by the same amount of movement LR according to the amount of operation of the trackball by a user, on the screens of Figs. 17 and 18. Thus, the amount of movement LR of ROI is the same with respect to the same amount of operation of the trackball between in Figs. 17 and 18, resulting in a good operability of the trackball for users.

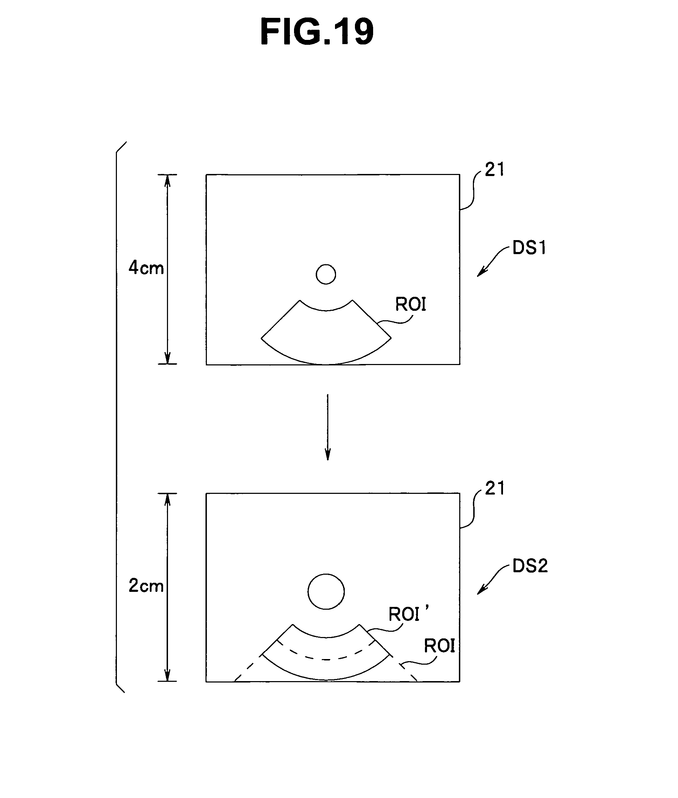

Moreover, when the display range of ROI is changed by the designation of a user, the size and position of ROI are automatically changed according to the change of the display range. Fig. 19 is a diagram to illustrate an example of the automatic adjustment of the position and size of ROI.

Suppose that the screen display state is changed from a state DS 1 to a state DS2. In the state DS1 of Fig. 19, the display range is 4 cm, and an ROI is displayed so as to be contained in the display range. Upon the display range being changed to 2 cm, the original position of the ROI becomes a position indicated by a dotted line in the state DS2, and it becomes hard for a user to see the ROI. Accordingly, configuration is made such that after the display range is changed, automatic adjustment of ROI is performed such that the ROI is included in the display range after change, thus generating a new ROI'.

It is noted that the operation portion 4 includes an ROI reset button, and upon the ROI reset button being depressed, the position and size of the ROI returns to its initial state.

### 5. Other functions

### 5.1 Measurement function

Moreover, the ultrasound diagnostic apparatus 1 also has a measurement function based on an ultrasound image.

It is noted that upon "Measurement Function" being selected from within a predetermined menu screen by a user, the apparatus turns into a measurement mode, and a caliper mark is automatically displayed on the screen. As a result of this, since simply selecting the measurement function causes the caliper mark to be displayed on the screen without the need of selecting measurement kinds, the user can easily recognize that the state of the apparatus is in a measurement mode only by viewing the screen.

### 5.2 Comment input function

The ultrasound diagnostic apparatus 1 also has a comment input function.

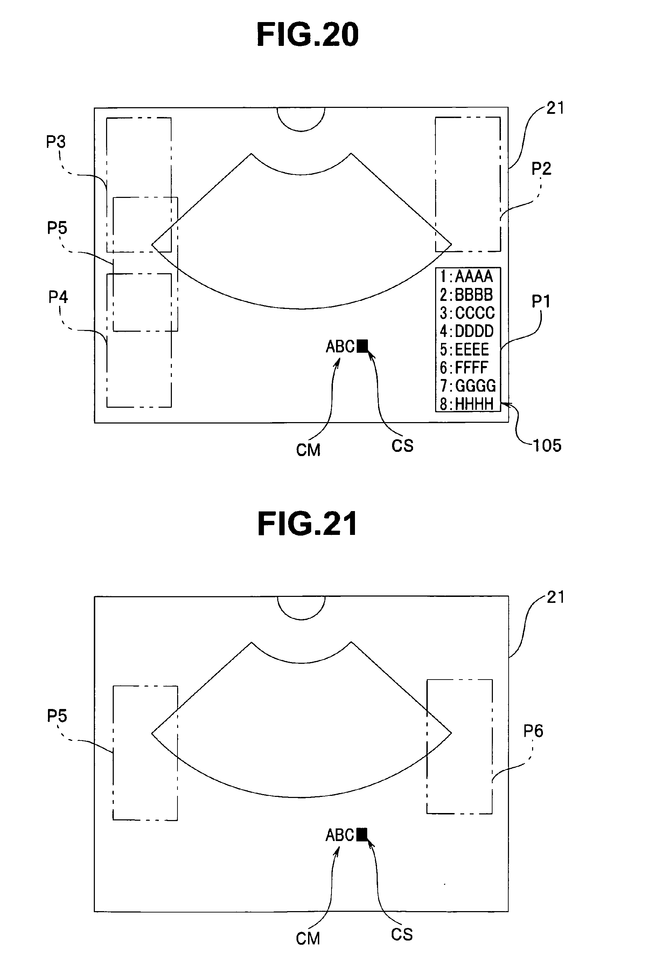

It is configured that a user can freely add comments on the screen on which an ultrasound image is displayed. Fig. 20 is a diagram to illustrate the case when a comment is inputted on the screen.

A user moves and sets a cursor to and at any position on the screen, and can freely write a comment at the position of the cursor. Since frequently used words are included in a comment, when a comment input mode is entered, an annotation list that displays such words in a list is displayed on the screen.

In Fig. 20, a comment CM of "ABC" is written at a desired position in the screen 21. While characters are inputted at the position of the cursor CS indicated by a rectangle, an annotation list display section 105 is also displayed on the screen. The annotation list is automatically displayed when a comment mode is entered, but it may be set that the list is not displayed automatically. When a user wants to input a word in the annotation list display section 105 at the cursor position, the user can designate a number thereby causing the word corresponding to the number to be written.

It is noted that the position of the annotation list display section 105 is configured to be changeable in the screen 21. The position of the annotation display section 105 is movable to a predetermined plurality of positions. Although the position of the annotation list display section 105 is at a lower right position P 1 in Fig. 20, it can be changed by a predetermined operation to an upper right position P2, an upper left position P3, or a lower left position P4 as shown by a two-dot-chain line. For example, when a user wants to change the position of the annotation list display section 105, the display position is changed in order among four positions in such a manner as P1, P2, P3, P4, P1, P2,.. every time the button is depressed. Therefore, the user can move the annotation list display section 105 to a desired position.

Moreover, the change pattern of the display position may not be the above-described pattern, that is, the position pattern of four points P1, P2, P3, P4, P1, P2,.., but may be a position pattern of three points P1, p2, and P5 of Fig. 20. In this case, the display position is changed in order among three positions in such a way as P1, P2, P5, P1, P2, P5,.. every time the predetermined button is pressed.

Further, Fig. 21 is a diagram showing another example of the display position of the annotation list display section 105. In Fig. 21, the display position is alternately changed between two positions in such a manner as P6, P7, P6, P7,.. every time the predetermined button is pressed. Thus, the display position of the annotation list display section 105 may be changed between tow positions as shown in Fig. 21.

Moreover, although a comment to be written on the screen can be written at anywhere on the screen, in order to prevent the comment from being superposed by a comment which has been written and the characters from becoming invisible, configuration is made such that the position of the cursor that designates the input position of comment cannot be set at a position within a half of the length (that is, height) of vertical direction (that is, longitudinal direction) from the center position in the horizontal direction of the characters.

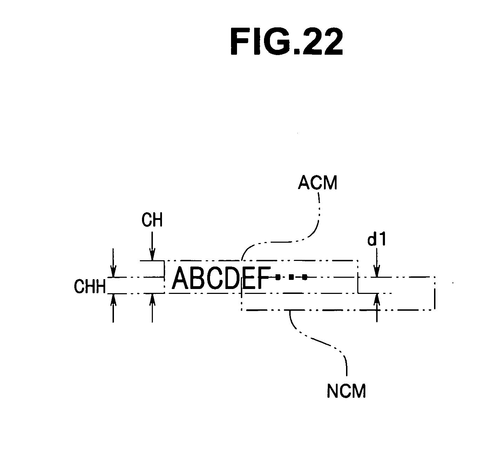

Fig. 22 is a diagram to illustrate the setup of a comment input position with a cursor. As shown in Fig. 22, when user wants to input a new comment in the vicinity of the region ACM of characters that have already been inputted, the comment input position is controlled such that even if the center of the cursor for designating the region NCM of new comment is within a half CHH of the height CH of a character, the center of the cursor cannot be set as the comment input position.

In Fig. 22, if a longitudinal difference d1 between the center position of the region ACM of characters already inputted and the center position of the cursor is within a half CHH of the height CH of character, the comment input position cannot be set to the position of the cursor. Therefore, the user cannot input new characters unless he/she moves the cursor to a position where there is no superposition of characters. By doing this way, it becomes possible to prevent the case in which comments are superposed with each other thereby becoming unreadable. Moreover, when characters are superposed in horizontal direction as well, superposition of comments may be prevented by configuring that a new character cannot be inputted unless the cursor is moved up to a position where there is no superposition of characters.

### 5.3 Transducer inspection function

Moreover, the ultrasound diagnostic apparatus 1 has a transducer inspection mode for the inspection of transducer.

Although the transducer inspection mode is not usually utilized by a user, an inspector can set the ultrasound diagnostic apparatus 1 to a transducer inspection mode when performing the maintenance of the ultrasound diagnostic apparatus 1 and the like.

Figs. 23 and 24 are diagrams showing examples of the screen to be displayed on the monitor 5 when the ultrasound diagnostic apparatus 1 is turned into a transducer inspection mode. As shown in Fig. 23, a plurality of ultrasound transducers are driven in order from an end along the scanning direction so as to generate sound ray, and if there is part that is not displayed in each sound ray obtained, it can be determined to be failure of the transducer. Here, an echo signal from each transducer is subjected to frame correlation and is averaged so that the image becomes more visible.

Moreover, Fig. 24 is a diagram showing an example in which element number and its value of signal strength not more than a predetermined threshold or a set threshold is displayed in a table format on the monitor 5 when determining a failure of the transducer. By seeing the table data of Fig. 24, the inspector of the ultrasound diagnostic apparatus 1 can discriminate at-a-glance the element of a signal level not more than the threshold, and its signal level. Thus, the inspector can determine the degree of failure or break of each element of a plurality of ultrasound transducers by using functions of Figs. 23 and 24.

### 5.4 Coping-with-failure function

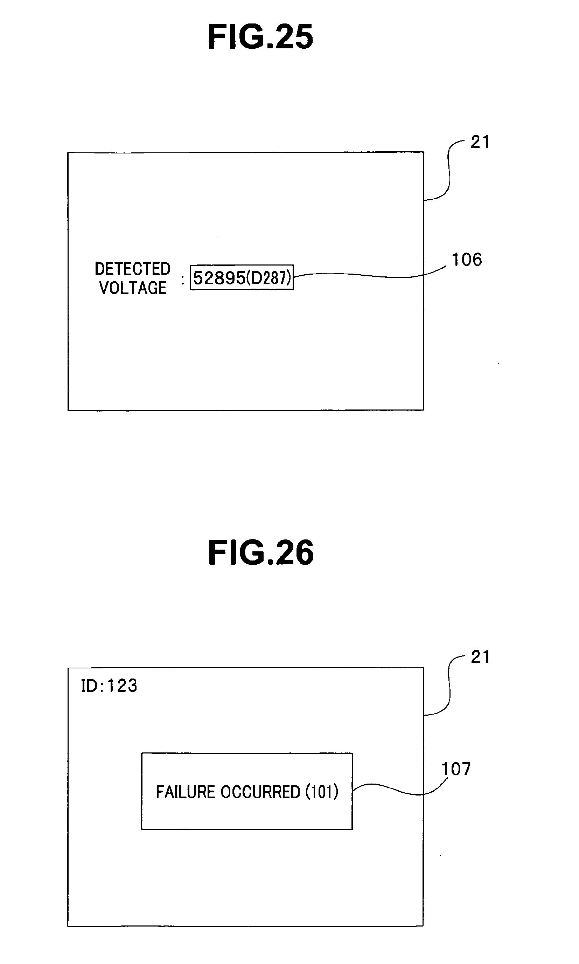

Further, the ultrasound diagnostic apparatus 1 has a function of displaying a detected voltage to check the voltage of the power supply at the time of power supply failure, etc. Fig. 25 is a diagram showing an example of the screen that displays a detected voltage of power supply. As shown in Fig. 25, a predetermined pop-up window 106 is displayed on the screen 21, and within the pop-up window, a detected voltage value is displayed by both a decimal number and a hexadecimal number.

It is noted that configuration may be such that either one of a decimal number and a hexadecimal number is displayed.

Moreover, when any message such as of failure etc. is displayed, not only the message but also a predetermined number or symbol will be added. Fig. 26 is a diagram showing an example of message display.

As shown in Fig. 26, a message, for example, a failure message is displayed in the pop-up window 107 on the screen 21.

While the message in this case is a message "A failure occurred", a predetermined number is added to the message. The number to be added has a meaning, which is predetermined such that, for example, the left end numeral of a three-digit number indicates a serious failure by "0", a medium failure by "1", a minor failure by "2", and an operation error by "3". By watching the added number when a message is displayed, a user or a maintenance worker can understand the meaning of the message, in this case, the site, level or content of failure.

Moreover, while an ID for identifying a patient is displayed on the screen, the font thereof is highlighted to prevent erroneous recognition. In the upper left of Fig. 26, there is a display indicating that the patient ID is "123". When fonts of each numeral "1", "2", "3" are displayed on the screen in a display "123", each numeral font is slightly displaced and superposedly displayed on the screen. As a result of this, characters are thickened thereby making it possible to improve the visibility of patient ID and to prevent erroneous recognition of patients.

### 5.5 Probe-related function

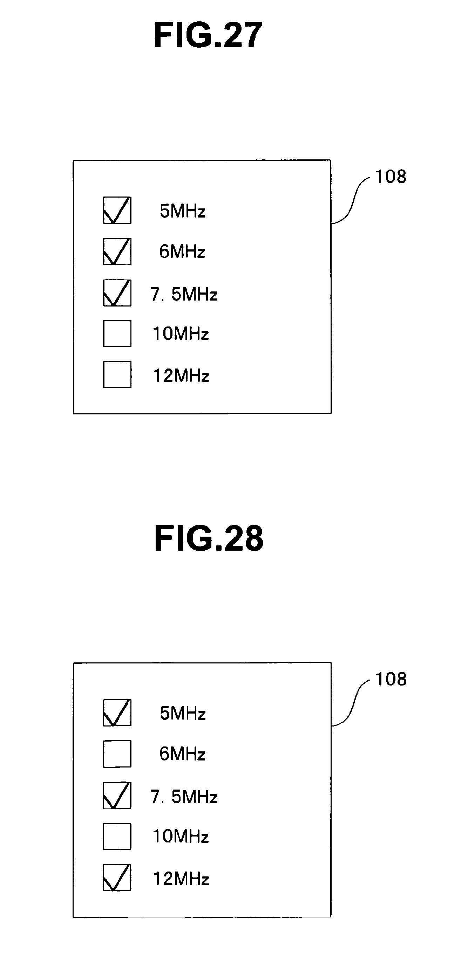

The driving frequency of probe can be set for each probe. Figs. 27 and 28 are diagrams showing examples of the setup screen of probe frequency. Upon operation to designate the driving frequency of probe being performed by a user, a setup screen as shown in Fig. 27 is displayed on the screen 21 as a pop-up window 108. If the user inserts a check mark in a checkbox for each frequency in the pop-up window 108a, the checked frequency is selected in a toggle manner every time a predetermined operation button, for example, a frequency key on the screen or of the operation portion 4 is depressed. In the case of Fig. 27, since a check mark is placed at 5 MHz, 6 MHz, and 7.5 MHz, the value of selected frequency displayed on the screen changes in order such as 5 MHz, 6 MHz, 7.5 MHz, 5 MHz, 6 MHz, every time the frequency key is pressed. In the case of Fig. 28, since a check mark is placed at 5 MHz, 7.5 MHz, and 12 MHz, the value of set frequency changes in order such as 5 MHz, 7.5 MHz, 12 MHz, 5 MHz, 7.5 MHz, every time the frequency key is pressed.

Therefore, it is possible to make it easier for a user to designate the driving frequency of each probe by the pop-up window 108, and to select the frequency that is desired to be set or changed from among designated frequencies by pressing a predetermined key.

When the ultrasound diagnostic apparatus 1 is not compatible with an ultrasound endoscope 2 or a probe 2a thereof which is to be connected to the ultrasound diagnostic apparatus 1, processing such as displaying a message "incompatible probe" on the screen 21, supplying no ultrasound signal to the probe, or providing no screen display is performed. For example, whether or not the probe 2a is compatible is determined by the identification information of the probe 2a, that is, the probe ID. Upon the ultrasound endoscope 2 being connected to an ultrasound observation apparatus 3, the information of 8-bit or 32-bit probe ID in a storage section provided in the probe 2a is read, and determination is made on whether or not the ultrasound observation apparatus 3 is compatible with the ultrasound endoscope 2 based on the probe ID.

### 5.6 Others

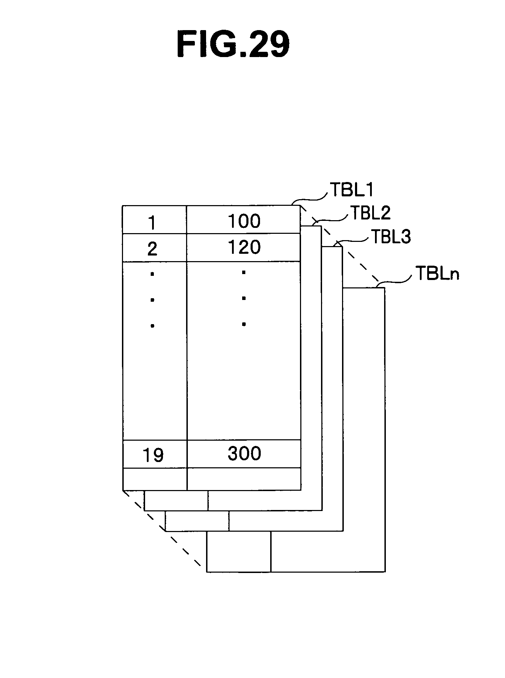

In the present embodiment, a gain setup table of B-mode is provided for each scope and probe in the ultrasound diagnostic apparatus 1. Fig. 29 is a diagram showing an example of a gain setup table of B-mode. As shown in Fig. 29, the ultrasound diagnostic apparatus 1 stores and retains the correspondence between the set values and display values of hardware as table data for each ultrasound endoscope and each probe in the storage section 14. Therefore, the ultrasound diagnostic apparatus 1 can change the gain of B-mode for each ultrasound endoscope and each probe.

Moreover, the ultrasound diagnostic apparatus 1 has a function of measuring a dynamic range of an electronic endoscope. Upon execution of the function, the ultrasound diagnostic apparatus 1 is configured to be able to drive an ultrasound transmission/reception circuit section 6 with a transmission power being set to 0 (zero) and a reception delay value being set to 0 (zero) as well.

Further, the ultrasound diagnostic apparatus 1 has a function of returning all of various parameters to default values. This function can be executed by the selection from a service menu or by a so-called secret command. Therefore, a user can return all of various parameters to default values by using a secret command based on, for example, a combination key operation.

Moreover, configuration is made such that the serial number of the ultrasound diagnostic apparatus 1 can be registered in an initial file (ini file) that is stored in the storage section 14. Therefore, it is possible that the serial number of the ultrasound diagnostic apparatus 1 is displayed on the screen of the observation monitor 5.

Furthermore, the ultrasound diagnostic apparatus 1 has a function of saving data obtained by sensitivity quantification software etc. in the storage section 14 in CSV format so as to be usable for various data processing software.

### 6. Decision processing of the size and position of ROI

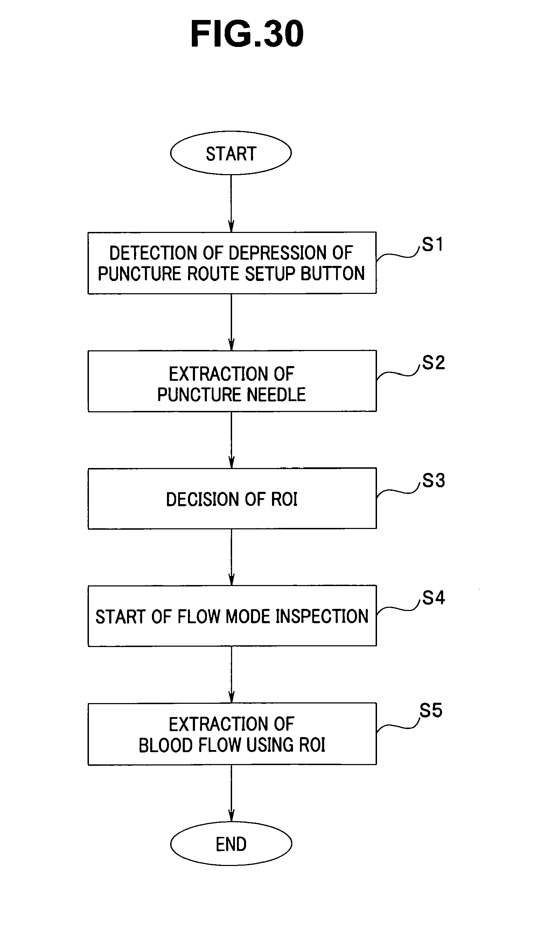

Next, the decision processing of the size and position of ROI according to the present embodiment will be described. Fig. 30 is a flowchart showing an example of the flow of processing including the decision processing of the size and position of ROI. The processing of Fig. 30 is executed before an inspection by using a puncture needle, and the like are performed.

Upon a predetermined button, specifically a puncture route setting button, among various buttons provided in the operation portion 4 being depressed by a user who is a physician after the ultrasound diagnostic apparatus 1 is activated, the ultrasound diagnostic apparatus 1 executes an ROI decision processing of the size and position of Fig. 30. It is noted that configuration may be such that a predetermined processing is executed by a predetermined operation not on a predetermined button, with respect to the operation portion 4. The processing program of Fig. 30 is stored in the storage section 14 in the ultrasound diagnostic apparatus 1, and the processing of Fig. 30 is executed by the CPU of the ultrasound diagnostic apparatus 1 reading out and executing the program.

The user wants to confirm if there is no blood vessel at some point on the path of the puncture needle 2c to be punctured hereafter. Then, in the present embodiment, to cause a flow mode image of a region including the start point and the end point of the puncture path of the puncture needle 2c to be displayed on the monitor 5 for that confirmation, the user performs operation to depress a puncture route setup button for that purpose. That is, as a result of that operation, a command for executing the processing program for the confirmation is given to the CPU.

Referring to Fig. 30, first, the processing to detect whether or not a predetermined button, that is, a puncture route setup button is depressed is executed, and thereby detection of the depression is performed (step S1). As a result of the depression operation, a command that causes a flow mode image display region, in which a flow mode image of a region including the start point and the end point of the puncture path of the puncture needle 2c is displayed, to be displayed on the screen of the monitor 5 is inputted to the CPU. Thus, the CPU makes up a command reception section for receiving the command.

Upon such button depression being detected, extraction processing of the puncture needle 2c is executed (step S2).

When after depressing the predetermined button of step S1, the user causes the puncture needle 2c to protrude from the distal end portion in, for example, water or a phantom, and performs predetermined button operation in the state of protrusion, an extraction processing of the puncture needle 2c by image processing is executed in step S2. The extraction processing of puncture needle will be described later in detail.

With the puncture needle 2c being protruded from the distal end portion of the probe 2a, and upon the extraction processing of puncture needle from an ultrasound image being ended, a decision processing of ROI is executed (step S3). Since as a result of the decision processing, the size and position of ROI is decided, an ROI that displays a flow mode image of a region including the start point and the end point of the puncture path of the puncture needle 2c is displayed. That is, the ROI is a flow mode image display region that displays a flow mode image of a region including the start point and the end point of the puncture path of the puncture needle 2c. Therefore, the CPU makes up a display control section that performs control to display such ROI on the screen. The decision processing of ROI will be described later in detail.

As so far described, upon ending of pre-processing of inspection and the like, an inspection by use of ultrasound, and the like becomes ready to be started.

Upon an ROI being decided, an inspection and the like is performed by a physician while observing blood flow. That is, a flow mode inspection is started (step S4), and extraction of blood flow by use of an ROI that displays a flow mode image of a region including the start point and the end point of the puncture path of the puncture needle 2c is performed (step S5).

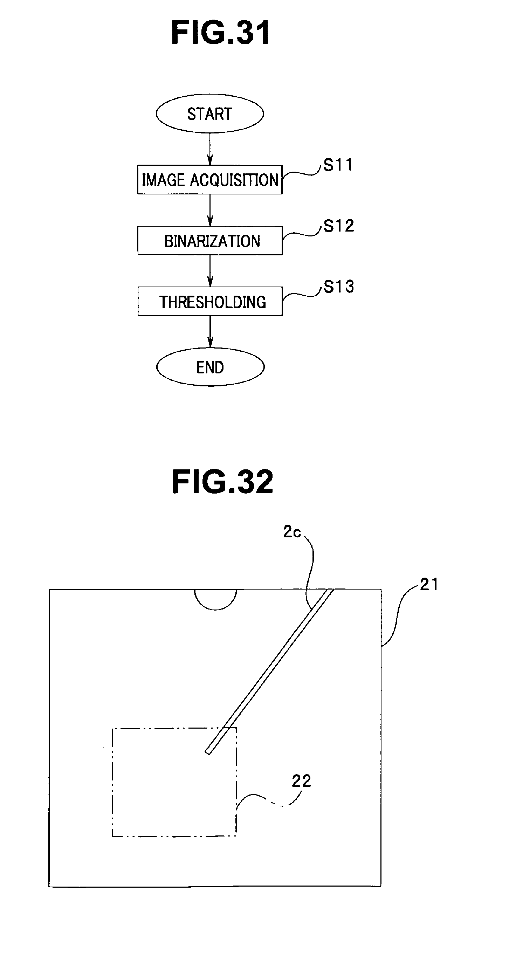

Next, the extraction processing of puncture needle in step S2 of Fig. 30 will be described. Fig. 31 is a flowchart showing an example of the flow of the extraction processing of the puncture needle of Fig. 30.

First, a predetermined button operation is performed with the puncture needle 2c being protruded from the distal end portion, and upon detection of the operation by the CPU, acquisition of ultrasound image is performed (step S11). For example, sponge etc. is disposed as a lesioned part in water etc., and the puncture needle 2c is protruded from the distal end portion of the probe 2a. Then, a still picture of ultrasound image is acquired with the puncture needle 2c being protruded.

Fig. 32 is a diagram showing an example of acquired image. As shown in Fig. 32, the puncture needle 2c extends toward a sponge 22. The angle at which the puncture needle 2c protrudes from the distal end portion of the probe 2a can be adjusted by the user with a raising mechanism (not shown) provided in the probe 2a. Thus, acquisition of ultrasound image is performed at a desired angle of the puncture needle 2c.

Next, binarization is performed on the acquired still picture (step S 12), and then thresholding that performs comparison with a predetermined threshold is performed (step S13). By extracting only predetermined image data from the binarized image data, it is possible to extract only the puncture needle 2c from the image. As so far described, an image of the puncture needle 2c is generated by image processing.

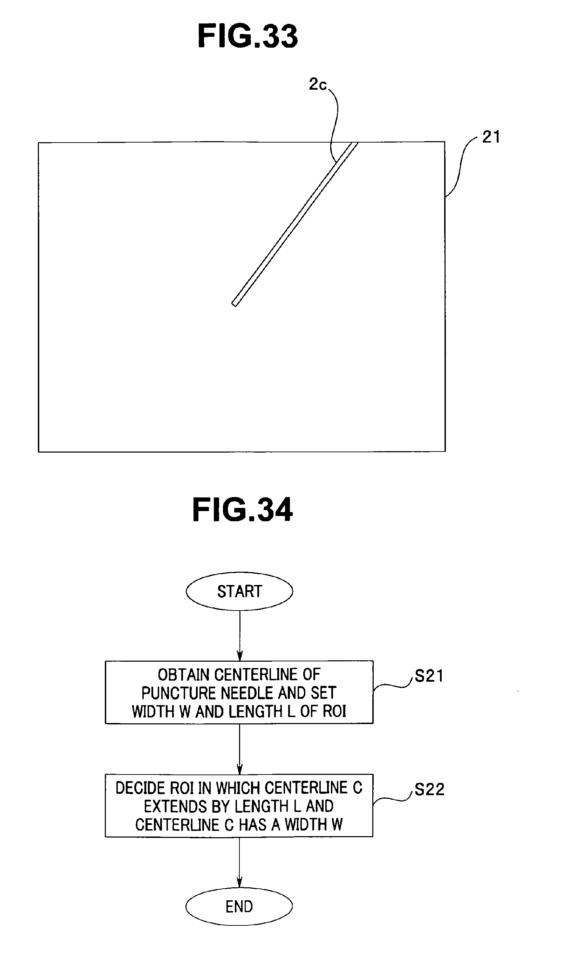

As a result, as shown in Fig. 33, an image of only the puncture needle 2c protruding from the probe 2a is obtained. Fig. 33 is a diagram showing an example of the generated image of the puncture needle.

Next, the decision processing of ROI of step S3 in Fig. 5 will be described. Fig. 34 is a flow chart showing an example of the flow of the decision processing of ROI in Fig. 30.

First, a centerline c of the puncture needle 2c in the image obtained as the result of the extraction processing of the puncture needle 2c of Fig. 31 is found, and then the width w of the ROI in the direction orthogonal to the centerline c, and the length L of the ROI extending from the distal end portion of the puncture needle 2c are decided (step S21).

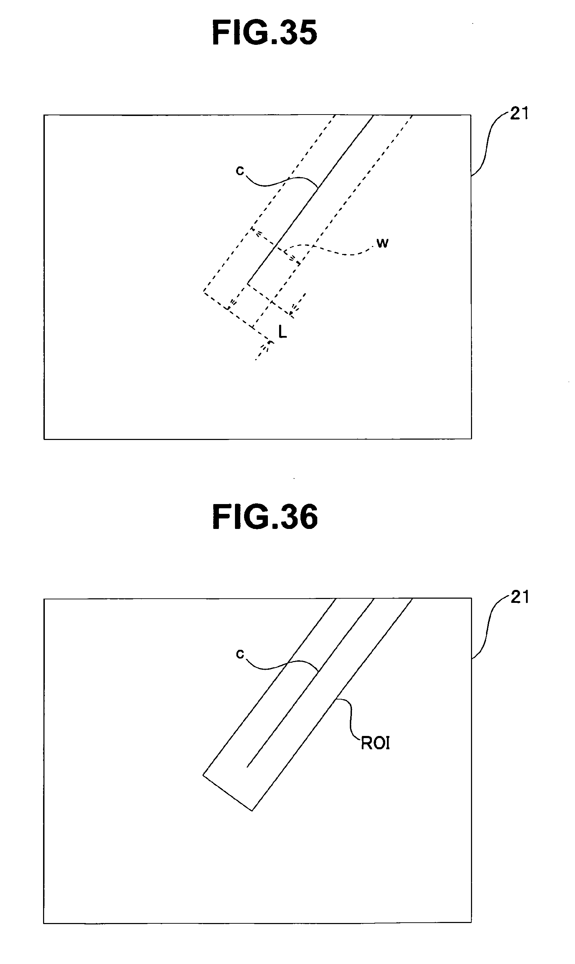

Fig. 35 is a diagram to illustrate the centerline c of the puncture needle 2c, and the width and length of the ROI. After the processing to find the centerline c of the puncture needle 2c is performed, the width of the ROI in the direction orthogonal to the found centerline c is decided. The value of the width w is preset by a user etc. and is stored in the storage section 14. Further, the amount of extension extending in the axial direction of the centerline c from the distal end of the centerline c, that is, a length L is decided. The value of the length L is also preset by a user etc. and is stored in the storage section 14.

Then, the CPU decides an ROI of an elongated shape having a width w and a length extending by a length L (step S22). That is, an ROI of a size having a width w and a length extending by a length L is decided at a position including an image of the puncture needle 2c.

Fig. 36 is a diagram showing a decided ROI. An ROI having a width w of Fig. 36 and a length L extending from the distal end of the centerline c is determined as an ROI at the time of puncture. The ROI of Fig. 36 is formed into an elongated shape, which includes the vicinity of the start point to the vicinity of the end point of puncturing of the puncture needle 2c, so as to include a puncture needle image.

As so far described, an ROI as a flow mode image display region during puncture is generated based on a puncture needle image of the puncture needle 2c through image processing by the CPU which is a display control section.

Then, the decided data of ROI is outputted from the control section 13 to a Region-Of-Interest setup section 11, and consequently the ROI image of Fig. 36 is displayed on the screen 21 under the control by the CPU.

Since a flow mode image is displayed in the ROI region during puncturing, the user can confirm whether or not a blood vessel is present in the region including the start point and the end point of the puncture path. That is, the ROI of Fig. 36 is a flow mode image display region during puncturing, in which a flow mode image of the region including the start point and the end point of the puncture path of the puncture needle 2c is displayed.

Thus, according to the ultrasound diagnostic apparatus of the present embodiment described above, since the decided ROI includes all the paths of the puncture needle 2c, that is, puncture routes, a user can confirm the presence/absence of blood flow without time and effort.

It is noted that since the region of ROI that is set up to include all the puncture routes as described above is narrower than in the case in which the region of ROI of a display form as shown in Fig. 15 is enlarged so as to include the puncture route, it is advantageous in that the CPU of the ultrasound diagnostic apparatus 1 does not need a high throughput, consequently leading to an increase in the frame rate of image.

### (Second Embodiment)

Next, a second embodiment of the present invention will be described.

Although, in the first embodiment, the decision processing of ROI is performed before an inspection using a puncture needle is performed since the probe has a raising mechanism etc. and the position of the puncture needle may change, there is also a case in which the probe has no raising mechanism etc. and the protruding angle and protruding length of the probe do not change. The second embodiment relates to a case in which the probe has no forceps raising mechanism and the protruding angle etc. of the puncture needle 2c does not change, and data of the position of ROI etc. is stored in the storage section in advance so that an ROI during puncturing is generated by using the stored data of ROI.

It is noted that since the ultrasound diagnostic apparatus of the present embodiment has a similar structure to that of the ultrasound diagnostic apparatus of the first embodiment, and also has the system configuration, various functions, etc. which are described in the first embodiment, description of the same components, functions, etc. is omitted and only different points will be described.

In the present embodiment, for example, the position and shape of the ROI shown in Fig. 36 is prestored as data in the storage section 14, and the prestored data of ROI is read out as initial position data at the time of puncturing to generate an ROI by using the read-out data of ROI.

It is noted that when the data of ROI is preset, the shape of ROI as a flow mode image display region may not be an elongated shape as in Fig. 36, but may be a conventional sector shape.

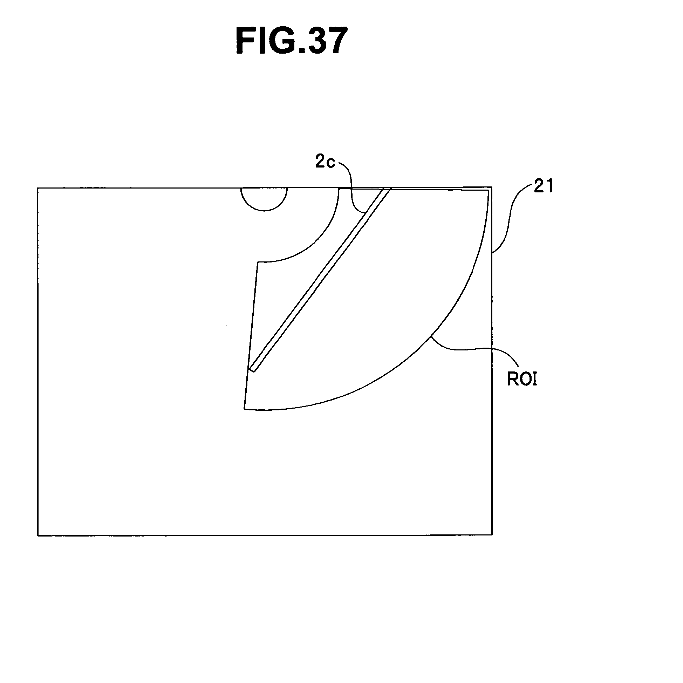

Fig. 37 is a diagram showing an example of a sector-shaped ROI. As shown in Fig. 37, the position, angle, etc. of the sector is adjusted so as to include all the routes of puncture needle. That is, the ROI of Fig. 37 includes from the vicinity of start point to the vicinity of end point of puncturing of the puncture needle 2c. The ROI of Fig. 37 is set in an approximately 3 o'clock direction with respect to the ROI of normal position in Fig. 15.

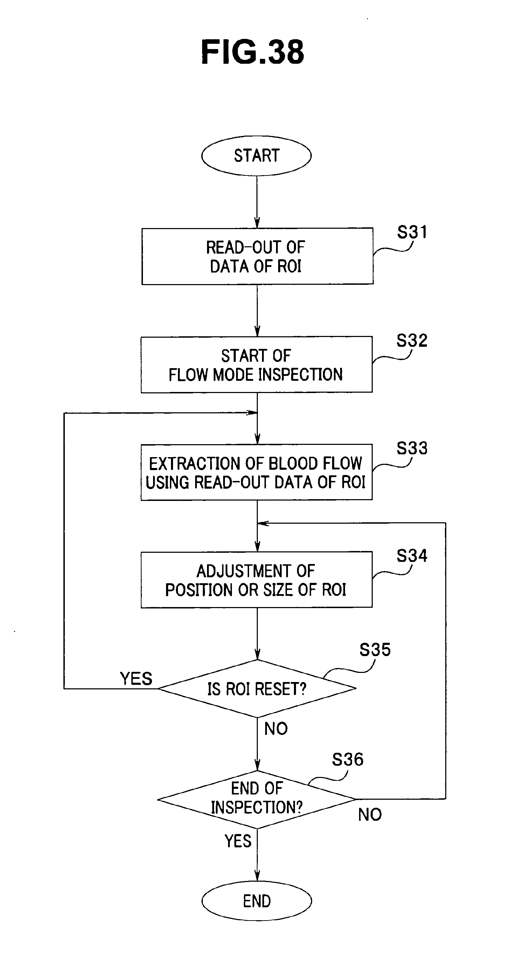

Fig. 38 is a flowchart showing an example of the flow of processing including the decision processing of ROI of an ultrasound diagnostic apparatus in the present embodiment.

First, when an inspection using the puncture needle 2c etc. is performed, the CPU of the control section 13 in the ultrasound diagnostic apparatus 1 reads out the data of ROI stored in the storage section 14 (step S31).

Then, an inspection while observing blood flow, that is, a flow mode inspection is started in the ultrasound diagnostic apparatus 1 (step S32), and extraction of blood flow is performed in the ROI decided by using the read out data of ROI (step S33).

A user may perform the adjustment of the position or size of ROI while performing the flow mode inspection as well (step S34), after the confirmation of the presence/absence of blood flow. For example, the user may want to see an image by a normal ROI, and also to adjust the position of ROI after having punctured the puncture needle 2c.

Further, after the ROI has been adjusted and moved away, the user may want to return the position of ROI to the original position of ROI, that is, the position of ROI based on the data of ROI stored in the storage section 14. Therefore, configuration is made such that the ROI can be returned to its original position.

Based on the performance of a predetermined button operation, etc., determination is made on whether or not a reset operation of ROI is performed (step S35). Upon performance of such resetting of ROI, the process becomes YES in step S35 and returns to displaying of the ultrasound image using the data of ROI read out in step S31.

If the process becomes NO in step S35, determination is made on whether or not the flow mode inspection has ended. Then, if the inspection has not ended, the process becomes NO in step S6, returning to step S34, and if the inspection has ended, the process ends.

It is noted that configuration may be made such that an ROI of normal position is displayed by performing a predetermined operation after an ROI based on the read out data of ROI is generated and the confirmation of the presence/absence of blood flow is finished. For example, the display is changed to an ROI display of normal position as shown in Fig. 15 after the display form as shown in Fig. 37, by performing a predetermined operation.

As so far described, according to the present embodiment, a user can confirm the presence/absence of blood flow of all the puncture routes without time and effort by setting and storing ROI in advance, and then reading out and using the stored data of ROI at the time of the inspection by puncture needle or the like.

It is noted that although, in the above example, the shape of ROI to be preset is an elongated shape and a sector, the shape may be, without being limited to those shapes, a triangle, an ellipse, a trapezoid, etc.

Further it is noted that although in the above described example, the data of a single ROI, which is prestored and read out in and from the storage section 14, is used, configuration may be made such that the data of a plurality of ROIs, for example, data of two ROIs of Figs. 36 and 37 are prestored, and a user selects which of the ROIs is to be used.

In that case, after step S31 of Fig. 38, a step in which the user selects which of the ROIs to be used among a plurality of ROIs is added, and extraction of blood flow is performed by using the selected ROI in step S33.

As so far described, the present embodiment can also achieve similar advantages to those of the first embodiment.

The present invention will not be limited to the embodiments described above, and various alterations and modifications can be made without departing from the spirit of the present invention.

The present application is filed claiming the priority of Japanese Patent Application No. 2008-231312 filed September 9, 2008 as its basis, and the above described disclosure is incorporated by reference in the present description and claims.

## Claims

1. An ultrasound image display apparatus, comprising a display control section that performs control such that a flow mode image display region is displayed on a screen of an ultrasound image, wherein the flow mode image display region is for displaying a flow mode image of a region including a start point and an end point of a puncture path of a puncture needle that appears on the screen when the puncture needle is protruded from an ultrasound probe.

2. The ultrasound image display apparatus according to claim 1, further comprising a command reception section that receives a command to display the flow mode image display region, wherein
the display control section performs control such that the flow mode image display region is displayed on the screen upon reception of the command by the command reception section.

3. The ultrasound image display apparatus according to claim 1 or 2, wherein
the display control section generates the flow mode image display region by means of image processing based on a puncture needle image at the time of puncturing of the puncture needle.

4. The ultrasound image display apparatus according to claim 3, wherein
in the image processing, the flow mode image display region is formed into an elongated shape including the puncture needle image.

5. The ultrasound image display apparatus according to claim 1 or 2, wherein
the display control section generates the flow mode image display region based on flow mode image display region data that is prestored in a storage section.

6. The ultrasound image display apparatus according to claim 5, wherein
the flow mode image display region has a sector shape or an elongated shape in the prestored flow mode image display region data.

7. The ultrasound image display apparatus according to claim 5, wherein
a plurality of the flow mode image display region data are stored in the storage section, and
the display control section generates the flow mode image display region based on data selected from among the plurality of the flow mode image display region data.

8. A method for displaying an ultrasound image, comprising performing control such that a flow mode image display region is displayed on a screen of an ultrasound image, wherein the flow mode image display region is for displaying a flow mode image of a region including a start point and an end point of a puncture path of a puncture needle that appears on the screen when the puncture needle is protruded from an ultrasound probe.

9. The method for displaying an ultrasound image according to claim 8, further comprising receiving a command to display the flow mode image display region, and
performing control such that the flow mode image display region is displayed on the screen upon reception of the command.

10. The method for displaying an ultrasound image according to claim 8 or 9, wherein
the flow mode image display region is generated by means of image processing based on a puncture needle image at the time of puncturing of the puncture needle.

11. The method for displaying an ultrasound image according to claim 10, wherein
in the image processing, the flow mode image display region is formed into an elongated shape including the puncture needle image.

12. The method for displaying an ultrasound image according to claim 8 or 9, wherein
the flow mode image display region is generated based on flow mode image display region data that is prestored in a storage section.

13. The method for displaying an ultrasound image according to claim 12, wherein
the flow mode image display region has a sector shape or an elongated shape in the prestored flow mode image display region data.

14. The method for displaying an ultrasound image according to claim 12, wherein
a plurality of the flow mode image display region data are stored in the storage section, and
the flow mode image display region is generated based on data selected from among the plurality of the flow mode image display region data.
